(19) Europäisches Patentamt / European Patent Office / Office européen des brevets

(11) **EP 3 066 466 B1**

(12) **EUROPEAN PATENT SPECIFICATION**

(45) Date of publication and mention
of the grant of the patent:
**14.10.2020  Bulletin 2020/42**

(51) Int Cl.:
*G01N 33/493* (2006.01)     *G01N 21/64* (2006.01)
*A01B 77/00* (2006.01)     *A01B 45/00* (2006.01)
*A01B 79/00* (2006.01)     *A01C 21/00* (2006.01)

(21) Application number: **14856865.2**

(22) Date of filing: **04.11.2014**

(86) International application number:
**PCT/NZ2014/000226**

(87) International publication number:
**WO 2015/065206 (07.05.2015 Gazette 2015/18)**

(54) **APPARATUS AND METHOD FOR EXCRETA DETECTION AND TREATMENT**

VORRICHTUNG UND VERFAHREN ZUR DETEKTION UND BEHANDLUNG VON EXKREMENTEN

APPAREIL ET PROCÉDÉ POUR LA DÉTECTION ET LE TRAITEMENT D'EXCRÉMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **04.11.2013  NZ 61734213**

(43) Date of publication of application:
**14.09.2016  Bulletin 2016/37**

(73) Proprietor: **Pastoral Robotics Limited
Auckland 1050 (NZ)**

(72) Inventors:
• **BATES, Geoffrey James
Auckland 1050 (NZ)**
• **QUIN, Bertram Francis Charles
Auckland 1071 (NZ)**
• **PALMER, George Terry
Auckland 1071 (NZ)**

• **SIMPKIN, Raymond Andrew
Auckland 0626 (NZ)**

(74) Representative: **Dolleymores
9 Rickmansworth Road
Watford, Hertfordshire WD18 0JU (GB)**

(56) References cited:
**WO-A1-02/19809          WO-A1-2004/013253
WO-A1-2006/132549     JP-A- 2009 270 975
US-A- 2 721 101          US-A- 5 033 397
US-A- 5 760 694          US-A- 5 846 830
US-A1- 2006 089 260     US-B2- 8 823 519**

• **L. RODHE ET AL.: 'Detection of urine patches on
grassland with an electromagnetic induction
-based sensor' NJF SEMINAR 438, NJF REPORT
vol. 6, no. 7, 28 October 2010, pages 60 - 62,
XP055288631**

**Description**

**FIELD**

This invention relates to apparatus and methods for excreta detection and treatment.

The invention is particularly suitable for detecting areas of pasture which have recently been subjected to an excreta deposition, and subsequently treating those areas with a substance(s). The invention will be described in relation to such use.

**BACKGROUND**

Nitrogen is an essential nutrient in fertilisers and soils for the growth of pastures. However, nitrate leaching (and gaseous nitrogen losses) are of significant concern to the dairy industry.

Nitrate leaching is a naturally occurring process, and occurs when nitrate leaves the soil in drainage water. Nitrate is soluble and mobile. There is no problem when it is within the root-zone of plants or pasture, but once it gets into the ground water and/or other fresh water bodies, it becomes an environmental pollutant.

Nitrogen is a significant constituent in the urine of dairy cows and is therefore returned or recycled to pastures when cows urinate.

Dairy cows deposit high rates of nitrogen in the form of urea (500-1000kgN/ha) via urine deposition. These quantities are far too large for the soil under the deposition to absorb, or for the pasture to be able to utilise it all.

Once an area of pasture has been soaked in the urine of a cow, the nitrogen in the urine (urea) transforms very quickly into ammonium-N, and then into nitrate-N. Because nitrate-N is water soluble, that which is not taken up by plant roots is leached into waterways, causing unwanted environmental effects.

In addition, much of the ammonium-N can be converted to greenhouse gases, ammonia and nitrous oxide. As a consequence, there is a significant loss of nitrogen from fertilisers, typically in the range of 20-100kgN/hectare annually.

A number of methods have been contemplated by scientists to minimise losses of nitrogen. These methods include devices to spread the urine over a larger area of the pasture; breeding cows with smaller bladders so that they urinate more frequently (and therefore over a larger total area); and producing grasses, clovers and other feeds that contain less nitrogen, so that a smaller amount of nitrogen is consumed by cows. However, none of these methods has yet proved to be a feasible or commercially feasible way of overcoming the problem.

A feasible method of overcoming the problem is to spread a nitrate inhibitor or a urease inhibitor on pastures. The main function of a nitrate inhibitor is to reduce the rate of conversion of ammonium-N into nitrate-N. The main function of a urease inhibitor is to reduce the rate at which urea-N is converted to ammonium-N. Either way, pastures have more time to absorb the nitrogen in the urine, before the nitrogen is converted to nitrate, which will then leach out of the soil very quickly.

However, it is generally not economically feasible, or otherwise practical, to blanket-spread a nitrate inhibitor and/or urease inhibitor over entire pasture areas. Instead, it would be significantly more practical and/or cost and time effective to only spread inhibitors at locations where a cow has actually urinated.

In this regard, it has been demonstrated in several studies* that the treatment of the relatively small areas of pasture recently affected by urine deposition ("urine patches"), before the urea-N in the urine has been converted to ammonium-N and subsequently to nitrate-N, can greatly reduce total nitrogen losses. However, because of difficulties in locating and/or treating individual urine patches, most existing methods designed to reduce losses still involve the impractical method of treating the entire grazed pasture with an inhibitor(s), despite the fact that at any one time, only 2-5% of the pasture area on a farm is likely to contain urine-N that has not already been converted to nitrate form.

NZ Patent No. 582798 incorporates a chlorophyll detection system which enables small areas of higher pasture growth, often the result of urine deposition, to be both excluded from fertiliser nitrogen application, and targeted for inhibitor application. However, the inherent difficulties with this technology are the high labour requirement, combined with the fact that if there is sufficient extra chlorophyll already present in the pasture above a urine deposition, it is likely that much of the urine-N deposited will already have been converted to nitrate form, as this is the predominant form of nitrogen taken up from the soil by pasture.

NZ Patent No. 254659 describes an apparatus which projects two wavelengths of light which are readily absorbed by the chlorophyll in plants. By analysing the light reflected, the apparatus is able to determine both the presence and size of the plant due to the

* For example, Di, H.J.; Cameron, K. C. 2002. The use of a nitrification inhibitor, dicyandiamide (DCD), to decrease nitrate leaching and nitrous oxide emissions in a simulated grazed and irrigated grassland. Soil Use and Management 18: 395-403, and Dawar, K.; Zaman, M, Rowarth, J.S.; Turnbull, M.H. 2012. Applying urea with urease inhibitor (N-(n-butyl) thiophosphoric triamide) in fine particle application improves nitrogen uptake in ryegrass (Lolium perenne L.). Soil Science and Plant Nutrition. Volume 58, Issue 3, 2012, pp 309-318 amount of light absorbed by the chlorophyll. The apparatus of NZ 254659 describes a method for the application of weed spray to plants which meet a certain user calibrated threshold of reflected chlorophyll absorbent light to reflected non chlorophyll absorbent light.

A disadvantage of the methods described in NZ 254659 and 582798 is that both rely on the plant having responded to the conditions of interest and producing more chlorophyll.

NZ Patent No. 506883 and also WO02/19809 A1 describe an animal-mounted supply of DCD and/or the urease inhibitor nBTPT in liquid, suspension or granulated forms. The action of the cow raising its tail to urinate or defecate triggers a small angle-sensitive valve on the tail to open, allowing a predetermined quantity of inhibitor to pass from the container, and through a tube, allowing deposition in the urine patch. While relatively cheap to produce, this device suffers from higher than desirable inhibitor application variance, and refilling and maintenance involvement by farm staff.

It may therefore be useful if it was possible to detect an environmental factor, such as a high urea or ammonium-N concentration in the soil, before the plant has noticeably responded to its presence and/or before it has significantly converted to nitrate-N.

The measurement of bulk soil conductivity has been used in agriculture as a method of detecting changes in the composition of soil. This has been applied to the detection of large depositions of urine*. However, the sensor they employed was not suitable for the detection of urine from individual animals, nor was it coupled to an autonomous method of treating the urine patches.

**[0019]** * See L. Rodhe, E. Salomon, and M. Gilbertsson, 'Detection of urine patches on grassland with an electromagnetic induction - based sensor', Proceeding at the NJF-Seminar 438: Jan 25, 2011.

A further prior art arrangement is known from US 5 033 397 and WO 2006/132549. These documents disclose apparatuses for measuring the amount of nitrate in the soil and apply a precise amount of fertilizer corresponding to the needs of the particular area. Although these documents foresee to deliver less fertilizer if cow urine is present, they do not foresee the use of inhibitors to reduce nitrate pollution.

## OBJECT

It is an object of the present invention to provide apparatus and methods for excreta detection and treatment which goes some way towards addressing one or more of the above problems or difficulties, or which at the very least provides the public with a useful choice.

## DEFINITIONS

Throughout this specification unless the text requires otherwise, the word 'comprise' and variations such as 'comprising' or 'comprises' will be understood to imply the inclusion of a stated integer or step or group of integers or steps but not the exclusion of any other integer or step or group of integers or steps.

## STATEMENTS OF INVENTION

According to one aspect of the present invention, there is provided an apparatus for travelling over a ground surface as recited by Claim 1.

According to a further aspect, there is provided a method as recited by Claim 11.

Further, preferred, features are presented in the dependent claims.

An advantage associated with the present invention is that it provides the user with an effective and practical method of identifying areas which have recently been subject to a deposition of excreta, and applying substances such as nitrification inhibitors or other chemicals to these areas. Furthermore, the invention also allows the user to apply other substances such as fertilisers to areas which are identified as not having been subjected to a deposition of excreta.

## PREFERRED EMBODIMENTS

The descriptions of the preferred forms of the invention to be provided herein, and with reference to the accompanying drawings, are given purely by way of example, and are not to be taken in any way as limiting the scope or extent of the invention.

## DRAWINGS

Fig 1:      is a perspective view of one possible embodiment of the apparatus of the present invention,

Fig 2:     is a plan view of the embodiment illustrated in Fig 1,

Fig 3:     is a view showing the nature of the electrodes illustrated in Figs 1 and 2,

Fig 4:     is a representation of a path that the apparatus may take around a paddock,

Fig 5a:    is a view of an alternative embodiment of the invention showing a first possible arrangement of the electrodes,

Fig 5b:    is a view of the embodiment of Fig 5a, with a second possible arrangement of the electrodes,

Fig 5c:    is a view of the embodiment of Fig 5a, with a third possible arrangement of the electrodes,

Fig 6:     is a view of an example not forming an embodiment of the invention which utilises non-contact electromagnetic sensing techniques, and

Fig 7:     is an equivalent circuit representation for detector coil and test object, for the example illustrated in Fig 6.

## DESCRIPTION OF PREFERRED EMBODIMENT

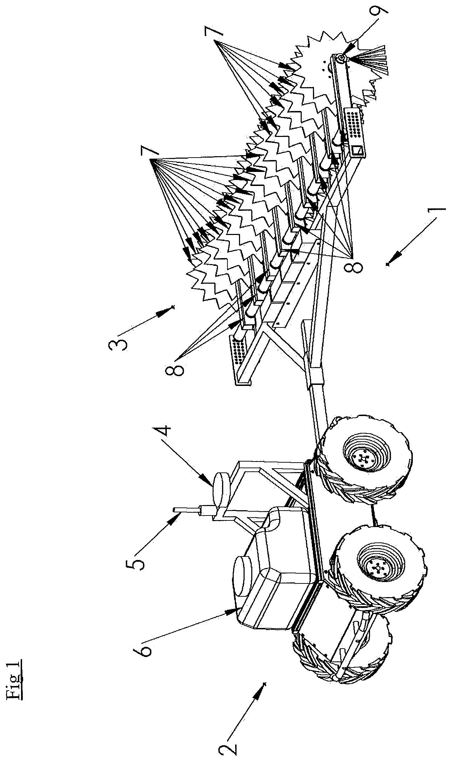

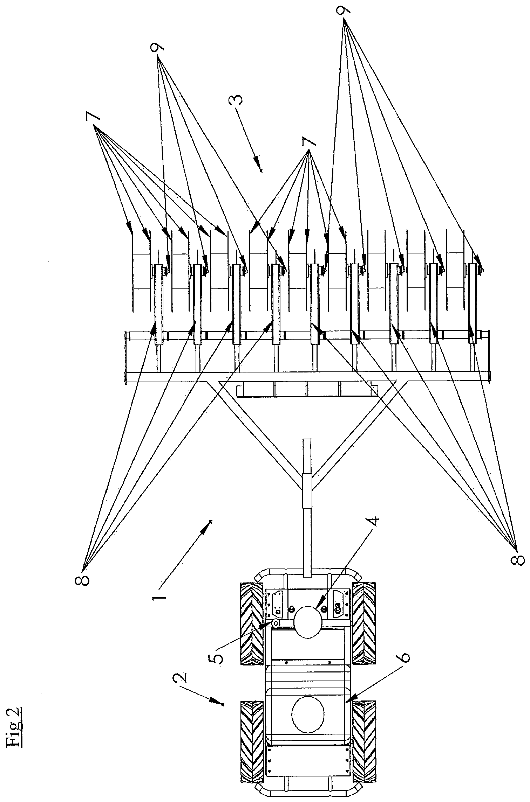

Having regard to Figs 1 and 2, there is depicted an apparatus for travelling over a ground surface, the apparatus being generally indicated by arrow 1.

The apparatus 1 is adapted to travel over an area of pasture and detect areas of the pasture which have had a (recent) deposition of urine, and subsequently treat those areas with either a nitrate inhibitor or a urease inhibitor (preferably a nitrate inhibitor).

The apparatus 1 is adapted to detect depositions of urine that are less than 72 hours old, but preferably less than 48 hours old.

Suitable nitrate inhibitors include (but are not limited to) ammonium thiosulphate and DCD. An example of a suitable urease inhibitor is nBTPT, better known by its trade name AGROTAIN.

The apparatus 1 includes a four-wheeled vehicle 2 which is adapted to tow a detection means, generally indicated by arrow 3.

The vehicle is automated and self-operating. To achieve this, the vehicle 2 includes a GPS unit 4. The GPS unit 4 includes RTK Differential GPS technology for navigation and/or mapping purposes.

The vehicle 2 also includes an antenna 5, which enables the vehicle 2 to be controlled remotely, as or when required or desired.

The vehicle 2 is battery powered (batteries not shown). In an alternative embodiment, the vehicle 2 may be petrol or diesel powered (or even solar powered).

The detection means 3 includes a plurality of electrodes in the form of substantially circular, yet spiky, rotatable metal discs 7. The metal discs 7 automatically rotate about their axes 8 as the detection means 3 is towed across the pasture by the vehicle 2.

The same battery or batteries that power the vehicle 2 also electrically power the discs 7 (the electrical connections from the vehicle to the detection means are not shown).

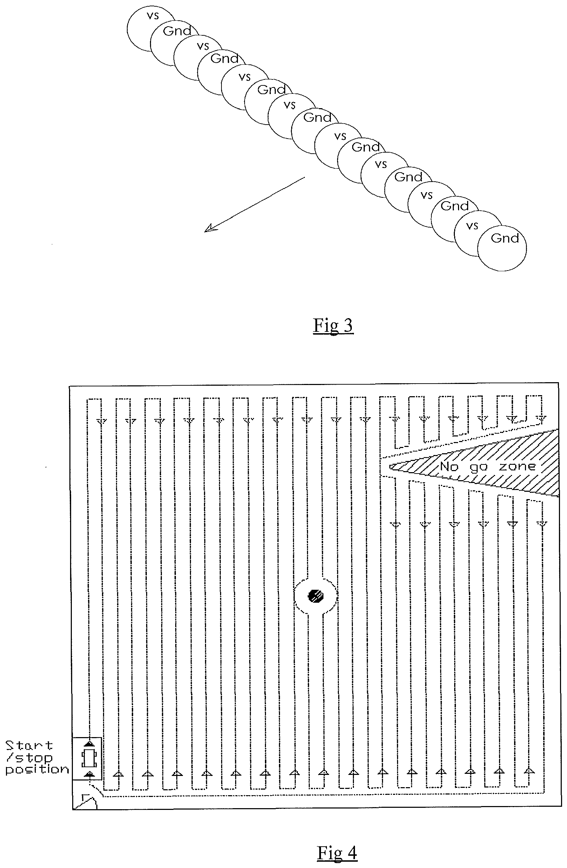

Fig 3 illustrates the electrical nature of the discs 7, where VS stands for Voltage Source and GND stands for Ground.

Fresh cattle urine contains materials which are highly conductive, relative to many soils. Hence, and with respect to the embodiment illustrated in Figs 1, 2 and 3, the presence of urine on the pasture surface (or sub-ground surface) may be detected by measuring an electrical current flow at or along the discs 7, which are in contact with the pasture surface.

Or in other words, the conductivity of the electrodes (discs 7) to the ground as measured by current flow is an indicator of the presence of urine.

In the embodiments illustrated in Figs 1, 2 and 3 we measure the current flow at all electrodes (discs 7) or at least at the positive or the negative discs 7 and look for large changes in current flow or high current flow relative to a running average or a fixed switch point. The VS and GND may be any value but are preferably 12 or 24VDC (there may be legal/safety reasons not to go above 50V).

In an alternative embodiment an AC current, and not a DC current may be used, as AC generally gives you much better electrode (disc 7) life.

The apparatus 1 also includes treatment means for treating each urine patch detected with the nitrate inhibitor.

The treatment means includes a 20Ltank 6 mounted on the vehicle 2, the tank 6 being filled with a nitrate inhibitor (in liquid form). The tank 6 is in liquid or fluid communication with a number of spray nozzles 9. Having regard to Fig 2, there is one spray nozzle 9 associated with a approximately every pair of discs 7.

The apparatus 1 also includes an electronic control means (not shown) for analysing the current flow or changes in current flow between the discs 7, and according to pre-programmed criteria, the control means may determine which areas of pasture have recently had a deposition of urine. Once the control means determines that the detection means 3 has identified and/or located a urine patch, the control means automatically and immediately instructs the relevant spray nozzle 9 to treat the urine patch by spraying an amount of the nitrate inhibitor.

For example, if the changes in current flow between the pair of electrodes (discs 7') indicated to the control means that the area of pasture immediately below the pair of electrodes 7' had received a recent deposition of urine, then the control means automatically and immediately instructs the spray nozzle 9' to treat this area by spraying it with the nitrate inhibitor.

Hence, as the apparatus 1 automatically makes its way around a paddock or pasture, the apparatus 1 is able to treat each urine patch detected with the nitrate inhibitor. An advantage of such a system is that a farmer does not need to apply blanket coverage of a nitrate inhibitor to the entire paddock or pasture, with resultant savings in cost, time and materials.

Fig 4 illustrates how the apparatus 1 may follow a predetermined path around a paddock or pasture, and shows the start and stop positions of the apparatus 1. Furthermore, Fig 4 illustrates how the apparatus 1 may automatically avoid obstacles such as rough and unsuitable terrain, noted in Fig 4 as "no go zone". The "no go zone" may be a swamp area or an embankment or a steep gully, etc.

Other features or embodiments of the apparatus 1 (and/or vehicle 2 and detection means 3) are detailed below.

The vehicle 2 may be adapted to move at any speed, although it is envisaged that a speed between 3km/hr - 10km/hr may be most suitable. If the vehicle 2 was adapted to move at 5km/hr it would cover 1.5 ha/hr. As a large herd of dairy cows (say 600-800) would normally strip-graze about 4 to 8 ha/day, the apparatus 1 will easily be able to treat the pasture grazed each day once the animals are moved off the area grazed.

The apparatus 1 may require a pre-programmed farm map preloaded with routes to follow based on given start points for each paddock. The farmer may move the apparatus 1 to a new paddock daily, and should preferably refill the 20L tank and swap the batteries. The apparatus 1 may be started by placing it in the correct position and orientation and pushing a single go button. Each paddock will have a predetermined path for the apparatus 1 to follow, with a fixed start-stop point as shown in Fig 4. As the apparatus 1 follows its path it will scan for, detect, and spray all recent urine patches.

All the paddock maps and the driving paths for each paddock will need to be loaded into the apparatus 1 on initial installation and whenever the farm boundaries, races or paddocks are changed.

The apparatus 1 may interface with existing products such as the TracMap system both for setup and where customers wish to record inhibitor application.

The apparatus 1 poses only a very negligible risk to humans, animals and fixed assets due to its low height and mass (less than 50 kg fully laden) and a low maximum ground speed (say, 5 km/hr). Safety is ensured by using GPS technology with sufficient allowance for the location accuracy coupled with sensors and design features to protect the apparatus 1 from harm. The apparatus 1 is a cost effective, medium-tech solution improving pastures, reducing nitrification rates and therefore nitrate leaching and gaseous nitrous oxide losses.

The addition of an extra spray tank to the apparatus 1 would allow the apparatus 1 to additionally apply fluidised urea (with urease inhibitor), trace elements and other additives as and where required. Application of urea will be linked to the urine detection so that the urea delivery will cut out over urine patches. The much-improved efficiency of fluidised, urease inhibitor-treated urea allows a greater area to be treated per tank-full than would granular urea.

The apparatus 1 may preferably include a pump (not shown) for pumping the liquid nitrate inhibitor (or urease inhibitor) from the tank 6 to, and out of, the spray nozzles 9.

In another embodiment, the liquid nitrate inhibitor may be supplied to the spray nozzles 9 under the force of gravity.

Whilst gravity feed has significant advantages, it would need to rely on valves, seals and active shutoff methods to ensure zero leakage whereas the pumped system will incorporate an anti-siphon design, without seals, that requires power at all times.

*Accuracy of location*

This will be achieved through the RTK Differential GPS technique and enhanced sensor fusion. Real time kinetic (RTK) GPS systems use the carrier signal as the data signal, ignoring the encoded information within it. Traditional GPS aligns the signals from satellites using the binary sequence, as the signal is encoded. The difficulty in RTK systems lies in aligning the signals accurately as every phase cycle of the carrier signal is undistinguishable. The receiver then compares its phase measurement to the phase measurement of a base station's correction signal. This technology allows users to achieve an absolute accuracy of up to 25 mm with tracking accuracies of 50 mm.

*Safety*

There are three critical levels of safety: people, animals and infrastructure. These may be assured via a fusion of multiple design features including covering a wide range of scenarios. Safety features are both inherent and active. Inherent safety features may include design features such as:

- Physical spaces which enable a person to climb onto the vehicle to avoid being crushed.

- Sufficient clearance to crawl underneath the vehicle.

- Design speed limits which ensure absolute maximum vehicle speeds are not above walking speeds.

- Access to emergency stop buttons from all sides of the vehicle.

Active safety systems may include:

- Proximity sensors capable of detecting safety concerns at a range that is greater than the worst case braking distance.

- Fusion of sensor information to ensure that accurate data is available irrespective of ambient conditions. Laser, vision, IR and sonar technologies all have optimum operating conditions and by fusing data it is possible to maximise the advantages of each technology.

- Automated and repeated calibration tests.

- Automated cleaning systems.

- Physical barriers linked to emergency stop switches, particularly at the front and back of the vehicle

- Fail safe stop modes, for example brakes that lock on in the event of a loss of power.

- Vehicle operation within acceptable environmental conditions only. Operation during rain fall is not required as the spreading of fertiliser and inhibitors is not recommended or allowable during these conditions. On board systems can measure ambient conditions and act appropriately.

The software of the control means may include the following safety algorithms;

- If there is any animal 20m in front, the apparatus 1 stops until the animal has moved

- If there is any animal in the same race or paddock and within 5 metres on any side, but not behind, the apparatus 1 may stop.

- If there is any animal within 1m on all sides of the apparatus 1, it may stop.

- No-go times can be programmed for races and other zones to avoid main periods of stock movement.

*Human-Machine Interface (HMI)*

The HMI is a web based interface that may communicate with the apparatus 1. The server can hold a map of the farm based on a farm survey. The map may contain paddock, race, gate, no-go zone, path and refilling/fuelling location data and can be updated by the user or through a service provider. The user may create action instructions as to the sequence of actions, for the apparatus 1 to undertake, these instructions may list the paddocks to visit in the desired order and the quantities of inhibitor etc to be deposited. When the sequence of instructions is complete, the apparatus 1 will return to the refill point to wait for further instructions. Whilst it may be essential that there is reception for data transfer at the refill station, the data transferred will be sufficient to allow the apparatus 1 to operate fully autonomously for periods when there may not be reception.

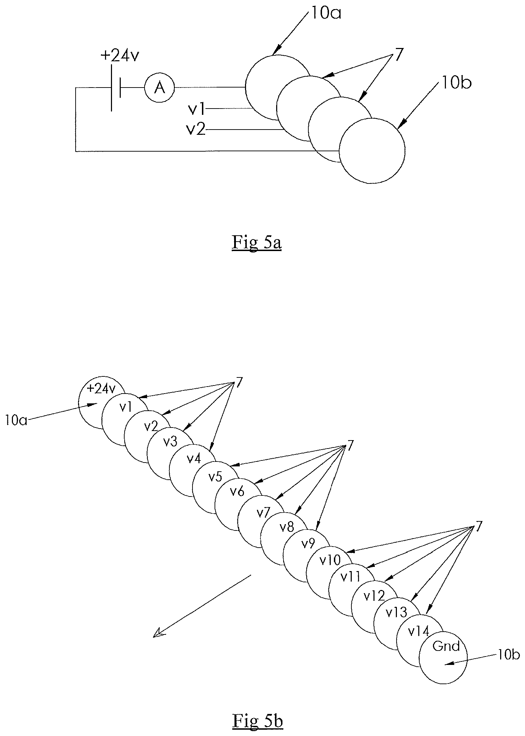

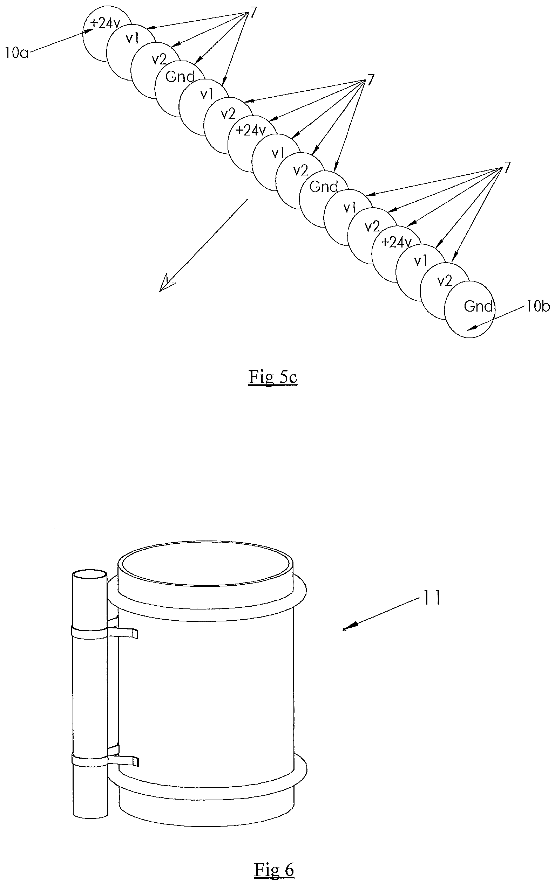

Having regard to Figs 5a, 5b and 5c, there are shown different embodiments of electrodes (discs 7) which detect the presence of urine on the ground surface (or sub-ground surface) by measuring the localised conductivity or resistivity of the soil of the ground surface (or sub-ground surface) and/or changes in the conductivity or resistivity of the soil.

As stated previously, fresh urine contains materials which are highly conductive, relative to many soils. As a consequence of the conductivity of the urine the presence of urine can be detected through measuring the localised conductivity or resistivity of the soil and/or changes in conductivity or resistivity of the soil.

The basic method of conductivity measurement is used in cropping and other applications as it gives you an indication of the soil structure. Essentially a current is applied between the two electrodes which in the case of our system are the outside conducting discs 10a and 10b as shown in Fig 5a. The current between the electrodes 7 is shown as A (for ammeter). The current can be measured and is constant at any point between the outer discs 10a and 10b. By measuring the potential difference at any location between the electrodes 10a and 10b, the conductivity of the soil near the surface can be measured. For example in Fig 5a, the potential difference is measured between the inner discs v1 and v2.

The conductivity of the soil is proportional to the current A divided by the potential difference (v1-v2), ie Conductivity = constant x A / (v1 - v2)

We do not need to know the constant to measure where changes in conductivity occur and for fresh urine patches the changes are quite significant. This method works very well as it is theoretically independent of the quality of the contacts until you get near the limits of conductivity when the signals approach the measurement limits of the system.

The advantage of this method is that the measurement is independent of the potential difference between either of the outer electrodes 10a and 10b and the soil. This potential difference is caused by the contact resistance and the current flow. As there is no significant current flow required to measure the potentials v1 and v2 the contact resistance for these inner electrodes v1 and v2 does not effect the conductivity measurement.

The conductivity at multiple locations can be measured in different ways, and two methods that can be used are described in relation to Figs 5b and 5c below.

What is unique is not that we are measuring conductivity but that we are using it to measure something on a micro scale (relatively) and not the macro scale used for bulk soil conductivity measurement when used in soil structure analysis.

Fig 5b illustrates current flows between outer electrodes (discs) 10a and 10b of an array. The arrow indicates the direction of movement of the array.

A 24VDC potential is shown between the outer discs 10a and 10b, and, for practical reasons, relating to safety, this potential shouldn't be higher than 50volts, though this is not a theoretical limit. AC current could also be used as it has less impact on the life of the electrodes though for initial development it is easier to use DC.

The potential is measured at each electrode 7 marked v1 to v14, although there is no limit to the possible number of electrodes 7. The potentials are measured by calculating the difference in potential between the electrodes 7 and applying the basic formula above.

The limitation of this method is that at some point the actual potential differences measured become too small and the measurement errors become too great. The wetter the soil the better the conductivity between the outside discs 10 and 10b, the better, because a higher current flows between the outside discs 10 and 10b, reducing the effective resistance between the outer discs 10 and 10b, and the soil, and therefore creating a greater potential difference that can be measured between each of the inner discs 7.

Fig 5c illustrates multiple electrode groups. More specifically, multiple groups of electrodes are created, a situation with five groups labelled A to E. The arrow indicates the direction of movement of the array.

Each of the five groups of electrodes A to E has to be read sequentially with the voltage supplies and grounds relating to the other sets isolated so as not to confuse the current measurements. In this manner the electrodes 7 have to be scanned and read one at a time which limits the speed of measurement compared to the first method.

This method has much less spatial resolution as the current source and ground electrodes do not measure potential but there is a much higher potential difference to measure across each of the current measuring electrodes. This greater potential difference allows this method to work with much less conductive, i.e. drier soils.

Figure 6 illustrates an example detection means not forming an embodiment of the invention which uses non-contact electromagnetic sensing techniques.

There is shown a magnetic induction sensor generally indicated by arrow 11.

The magnetic induction sensor 11 includes a single turn of copper tubing which is used as a transmit coil connected to a variable frequency oscillator. The time-varying voltage applied to the transmit coil creates a time-varying magnetic field that permeates the space around it. Offset from the transmit coil by a height approximately equivalent to the diameter of the transmit coil, is a single turn of copper tubing used as the receiver coil. This coil constitutes the detector element of the sensor and is connected in parallel with a fixed capacitor. The inductance of the receiver coil and the capacitance of the fixed capacitor create an electrically resonant circuit.

Table 1 below lists the design parameters of the sensor.

**Table 1: Sensor specifications**

| | |
|---|---|
| RX coil diameter (internal) | 315mm |
| Diameter of RX coil wire | 6mm copper tubing (OD) |
| Number of turns in RX coil | 1 |
| TX coil diameter (internal) | 315mm |
| Diameter of TX coil wire | 4mm copper tubing (OD) |
| Number of turns in TX coil | 1 |
| Fixed capacitance | 470pF polystyrene film capacitor |
| RX-TX coil vertical separation | 315mm |
| Distance from RX coil to test object plane | 50mm |
| Coil former | White PVC pipe, wall thickness 12mm |

When a material with dielectric and conducting properties is brought into the vicinity of the receiver coil, such as urine-contaminated soil, the resonant frequency and magnitude of the received voltage change. The change in resonant frequency and voltage are readily measured quantities and can be related to the properties of the nearby object of interest such as soil and/or urine. Thus, the presence of a urine patch can be so detected.

**Equivalent circuit representation of the sensor**

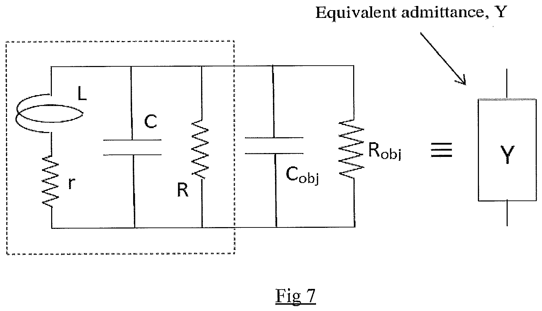

To describe the behaviour of the magnetic induction sensor, a useful and powerful description of the detector coil's properties is an equivalent circuit representation. Fig 7 shows such an equivalent circuit representation for detector coil and test object.

Components shown in the dotted outline are those of the detector coil circuit and those remaining are associated with the test object (subscript "obj").

In Figure 7, the inductance of the detector coil is denoted by $L$ with its RF resistance denoted by, $r$. The fixed capacitor connected in parallel across the coil is denoted by $C$. The parallel resistance across the coil is denoted by $R$. These three components represent the characteristics of the detector coil (shown enclosed in the dashed outline in Figure 7).

The inclusion of the parallel resistance, $R$, as part of the inherent coil circuit, was found to be necessary in order to correctly account for two contributions to the coil's behaviour, namely:

(i) The effect of the transmit coil on the receiver circuit. The transmit coil is itself a metallic object that lies within the sensitive region of the receiver coil. Therefore, the transmit coil looks like a conductive test object to the receiver circuit which can be modelled by a parallel resistance, $R$.

(ii) The non-deal behaviour of the fixed capacitor used.

Both of the above effects can be modelled by the inclusion of the parallel resistance, $R$, in the equivalent circuit.

The remaining capacitor, $C_{obj}$ and resistor, $R_{obj}$, represent the properties of the test object. Allowance must be made for the capacitive effect of the test object as well as its conductance to account for the effects of the test object's dielectric constant.

The equivalent circuit of Figure 7 can be analysed in the frequency domain to give an equivalent complex input admittance for the combined detector coil and test object denoted by the function $Y(\omega)$, where $\omega$ is the angular frequency of the applied magnetic field. The expression for $Y(\omega)$ in terms of its real and imaginary parts, $G$ and $B$, as a function of the circuit parameters is given below:

$$Y(\omega) = G + jB$$

where $G$ and $B$, respectively, are the conductance and susceptance of the equivalent input admittance, and are defined as:

$$G = \frac{1}{R} + \frac{1}{R_{obj}} + \left(\frac{r}{r^2 + (\omega L)^2}\right)$$

$$B = \omega(C + C_{obj}) - \left(\frac{\omega L}{r^2 + (\omega L)^2}\right) \quad (1)$$

Of particular interest are the equivalent circuit parameters of the test object in isolation, namely, $R_{obj}$ and $C_{obj}$, which can be combined to form the test object's complex admittance, $Y_{obj}$, as follows:

$$Y_{obj} = G_{obj} + j B_{obj}$$

$$(1a)$$

where in (1a):

$$G_{obj} = \frac{1}{R_{obj}} = \text{Equivalent conductance of test object } (1/\Omega)$$

$$B_{obj} = \omega C_{obj} = \text{Equivalent susceptance of test object } (1/\Omega)$$

In the following, the various parameters that must be measured are discussed and how they are related to the equivalent circuit parameters.

**Resonant frequency**

The equivalent circuit becomes resonant when $B = 0$. Using equation (1), this gives the following expression for the resonant angular frequency, $\omega_0$:

$$\omega_0 = \sqrt{\frac{1}{L(C + C_{obj})} - \left(\frac{r}{L}\right)^2}$$
$$\cong \sqrt{\frac{1}{L(C + C_{obj})}}$$

$$(2)$$

The approximation used in equation (2), that of ignoring the effect of the coil's RF resistance, $r$, is valid for most practical coils using copper wire. It can be seen from this expression that the test object can shift the resonant frequency compared to the case with no object present (for which $C_{obj} = 0$) via its capacitive effect.

It is apparent that if we make a measurement of the resonant frequency with no object present (the so-called 'open' case) then we can determine the coil's inductance since the fixed external capacitance $C$ is known.

Repeating the above for the case with a test object in the vicinity of the receive coil then allows the equivalent capacitance of the object, $C_{obj}$, to be found using the following expression which arises from (2):

$$\frac{C_{obj}}{C} = \left(\frac{f_0}{f}\right)^2 - 1 \qquad (2a)$$

where in (2a):

$f_0$ = Resonant frequency for 'open' case.
$f$ = Resonant frequency with test object present.

**Sensitivity, S**

When a test object is introduced into the vicinity of the receive coil, the main effect on the received signal is a reduction in the amplitude of the coil voltage at its resonant frequency. This is due to the increased dissipation of electrical energy in the test object by virtue of its conductivity and is represented in the equivalent circuit by the parallel resistance, $R_{obj}$.

We can define the system sensitivity to the object's conductivity as the fractional change in voltage at resonance using the 'open' case as reference. This parameter is readily measured.

In terms of the circuit parameters, the sensitivity, $S$, can then be expressed as:

$$S = Q_0 Z_0 \left( \frac{1}{R_{obj}} + \frac{rC_{obj}}{L} \right) \simeq \frac{Q_0 Z_0}{R_{obj}} \quad (3)$$

where in (3):

$$Z_0 = \sqrt{\frac{L}{C}} \text{ = characteristic impedance of receive coil with no object present.}$$

$Q_0$ = Quality factor of the resonant circuit with no object present.

The approximation shown on the right-hand side of (3) is valid since, in practice, $\frac{1}{R_{obj}} \gg \frac{rC_{obj}}{L}$.

Equation (3) shows that for a test object with given equivalent circuit parameters, the sensitivity is high when the product $Q_0 Z_0$ is large for the receive coil.

**Quality factor, $Q_0$**

The quality factor of the resonant circuit with no test object present (the 'open' case) denoted by, $Q_0$, can be defined in terms of the resonant frequency, $\omega_0$ and frequency bandwidth at the half-power points, $\Delta\omega$ as follows:

$$Q = \frac{\omega_0}{\Delta\omega} \quad (4)$$

The resonant frequency and bandwidth are readily measurable quantities if one sweeps the excitation frequency over a band centred on the resonance. For the equivalent circuit in Figure 2, and considering no object present, we can express $Q_0$ in terms of the circuit parameters as follows:

$$Q_0 = \frac{Z_0}{\left[ r + \left( \frac{Z_0^2}{R} \right) \right]} \quad (5)$$

In the absence of any Ohmic coil resistance, ($r = 0$), the limiting value for the Q-factor is $\frac{R}{Z_0}$.

The characteristic impedance, $Z_0$, depends on the value of fixed capacitance, $C$, and on the receiver coil inductance, which in turn depends on the coil geometry (typically, coil diameter, number of turns and wire radius) which can be independently controlled to a large extent.

**Summary of measurements required**

In summary, in order to detect the presence of a urine patch in soil, the following measurements must be made with the magnetic induction system:

1. Determine the resonant frequency, $f_0$, the Q-factor, $Q_0$, and output voltage at resonance, $V_0$ , of the system with no test object present (open case). These measurements constitute a calibration of the coil system and can be made by placing the sensor at a height well above the ground.

2. With a test object in place, such as soil with or without urine, determine the new resonant frequency, $f$, and the output voltage at resonance, $V$.

From these measured quantities, the real and imaginary parts of the test object's equivalent admittance, $G_{obj}$ and $B_{obj}$, respectively, can be determined from the following equations:

$$G_{obj} = \frac{1}{R_{obj}} \cong 2\pi f_0 C \left( \frac{V_0 - V}{Q_0 V_0} \right)$$

$$B_{obj} = 2\pi f C_{obj} = 2\pi f C \left[ \left( \frac{f_0}{f} \right)^2 - 1 \right] \tag{6}$$

It should be noted that the equivalent circuit parameters of the test object will be dependent on the distance between the receive coil and test object. This aspect, and how to correct for it, are discussed in the following.

Tables 3, 4 and 5 below show measured results for the detector coil voltage versus frequency at three different sensor stand-off heights above a urine-contaminated patch of soil, and the same patch of soil with no urine present. The sensitivity of both the resonant frequency and amplitude of receiver voltage at resonance is clearly visible with and without the urine present.

Table 3:
Measured results for urine patch on soil using stand-off height of 50mm

**Table 4:**
**Measured results for urine patch on soil using stand-off height of 95mm**
▲ No urine   ▲ Urine patch

**Table 5:**
**Measured results for urine patch on soil using stand-off height of 140mm**
▲ No urine   ▲ Urine patch

From the data in Tables 3,4 & 5, equivalent circuit parameters can be calculated using the expressions in equation (6). The aim is to calculate the equivalent conductance, $G_{obj}$ and susceptance, $B_{obj}$ for urine-contaminated and urine-free soil samples for each stand-off height. The results for $G_{obj}$ and $B_{obj}$ are shown in Figures 6 and 7, respectively.

From the results shown in Table 6 below, the effect of the urine on the soil manifests itself as an increase in equivalent conductance over and above that for urine-free soil as one would expect. The variation in equivalent conductance with stand-off height for urine-free and urine-soaked soil is similar for both cases with only a slight increase in the difference in conductance observed with decreasing stand-off height.

**Table 6:**
**Equivalent conductance at resonance, $G_{obj}$, versus stand-off height**

Table 7 below shows that the equivalent susceptance increases with decreasing stand-off height for both urine-free and urine-soaked soil, as expected, but at a noticeably accelerated rate for the urine-soaked soil compared with the urine-free case.

**Table 7:**
**Equivalent susceptance at resonance, $B_{obj}$ versus stand-off height**

If we now consider the ratio of the equivalent conductance to susceptance for each stand-off height, the graph shown in Table 8 below results. This figure shows that the ratio $G_{obj}/B_{obj}$ varies little with stand-off distance for the urine-free case, *but is highly sensitive to it for a urine patch.*

**Table 8:**
**Ratio of equivalent conductance to susceptance at resonance, $G_{obj}/B_{obj}$ versus stand-off height**

This means that, by computing this ratio, it is possible to distinguish a urine-free region from a urine-soaked one without having to accurately maintain a fixed sensor height above the soil.

To achieve this outcome, the sensor stand-off height should not exceed approximately 75mm, thereby maintaining a sufficient separation in values of $G_{obj}/B_{obj}$ for urine-free and urine-soaked soil.

For the urine-free case, the $G_{obj}/B_{obj}$ ratio could be evaluated at *any* stand-off height, including the 'infinite' stand-off distance given by the 'open' case. This would set the upper limit for this ratio as Figure 8 suggests.

Detection of a urine patch then proceeds by computing $G_{obj}/B_{obj}$ at each location over the paddock. Detection of a urine patch would then be deemed to have occurred if this value fell below a pre-defined threshold value, which could be expressed as a percentage of the upper limit value.

Furthermore, the results of Table 8 suggest that a smaller stand-off distance than the minimum of 50mm used for these measurements, would enhance the contrast between urine-soaked and urine-free soil.

An advantage associated with the present invention is that it provides the user with an effective and practical method of identifying areas which have recently been subject to a deposition of excreta, and (immediately or shortly thereafter) applying substances such as nitrification inhibitors or other chemicals to these areas. This results in the user (farmer) benefiting from lessor amounts of nitrate leaching, which is not only beneficial to the farmer's farm, but also for the environment as a whole.

Furthermore, the invention also allows the user to apply other substances such as nutrients or fertilisers to areas which are identified as not having been subjected to a deposition of excreta.

### VARIATIONS

While the embodiments described above are currently preferred, it will be appreciated that a wide range of other variations might also be made within the scope of the invention as defined by the appended claims.

### Claims

1. An apparatus for travelling over a ground surface, said apparatus including:

   a) detection means (3) for detecting an area of the ground surface (or sub-ground surface) which has had a deposition of excreta,

   b) treatment means (6, 9) for treating the area with at least one substance,
   wherein said detection means being adapted to detect the presence of excreta on the ground surface (or sub-ground surface) by measuring an electrical current flow at a plurality of electrodes which are in contact with the ground surface, **characterised in that** said electrodes are in the form of substantially circular and rotatable metal discs (7), wherein the ground surface is pasture and the excreta being detected and treated is cattle urine, and wherein the at least one substance includes a nitrate inhibitor and/or a urease inhibitor.

2. An apparatus as claimed in Claim 1, wherein the ground surface is pasture and the detection means is adapted to detect the presence of urine on the ground surface (or sub-ground surface) by measuring the localised conductivity or resistivity of the soil of the ground surface (or sub-ground surface) and/or changes in the conductivity or resistivity of the soil.

3. An apparatus as claimed in Claim 2, wherein the detection means includes:

   a) two outer electrodes in contact with the ground surface,
   b) at least two inner electrodes in contact with the ground surface, said at least two inner electrodes being positioned between the two outer electrodes and being electrically connected to the two outer electrodes,
   c) a power source for providing an electrical current flowing between the two outer electrodes (and therefore through the at least two inner electrodes),
   d) means for measuring the electrical potential between the at least two inner electrodes,
   e) control means for analysing the electrical potential between the at least two inner electrodes, whereby the localised conductivity and/or resistivity of the ground surface (or the sub-ground surface), may be determined, and as a result, any areas which have had a deposition of urine can be identified and/or located,
   the arrangement and construction being such that the control means subsequently instructs the treatment means to automatically treat areas with the substance(s) which are determined to have had a deposition of urine.

4. An apparatus as claimed in Claim 1, wherein the rotatable metal discs include spikes around the periphery thereof for engaging with and/or penetrating the ground surface.

5. An apparatus as claimed in any one of Claims 1 to 5, wherein the apparatus is a wheeled vehicle or is adapted to be towed behind a wheeled vehicle.

6. An apparatus as claimed in Claim 6, wherein the vehicle is operated by remote control.

7. An apparatus as claimed in Claim 6, wherein the vehicle is automated and self-operating.

8. An apparatus as claimed in any one of Claims 6 to 8, wherein the vehicle includes proximity sensors.

9. An apparatus as claimed in any one of Claims 1 to 8, wherein the apparatus includes a store of each of the at least one substance, each of said store(s) being provided with a distribution means to enable the at least one substance to be applied to each area of the ground surface where a deposition of excreta has been detected by the detection means.

10. An apparatus as claimed in Claim 9, wherein the distribution means includes one or more spray nozzles which are adapted to spray the at least one substance on each area of the ground surface where a deposition of excreta has been detected by the detection means.

11. A method for detecting an area of a ground surface (or sub-ground surface) which has had a deposition of excreta, and subsequently treating the area with a substance(s), **characterised by** said method including the step of utilising the apparatus as claimed in any one of Claims 1 to 10.

12. A method as claimed in Claim 11 for detecting and treating a recent deposition of cattle urine, namely within 72 hours of being deposited.

**Patentansprüche**

1. Eine Vorrichtung zu Überfahren einer Bodenoberfläche, wobei die Vorrichtung Folgendes umfasst:

   a) Erkennungsmittel (3) zum Erkennen eines Bereichs der Bodenoberfläche (oder des Untergrunds), auf dem Exkremente hinterlassen wurden,
   b) Behandlungsmittel (6, 9) zur Behandlung des Bereichs mit zumindest einer Substanz,

wobei

das genannte Erkennungsmittel so adaptiert ist, dass es das Vorhandensein von Exkrementen auf der Bodenoberfläche (oder im Untergrund) durch Messen eines elektrischen Stromflusses an einer Vielzahl von Elektroden, die mit der Bodenoberfläche in Kontakt sind, nachweist, **dadurch gekennzeichnet, dass** die genannten Elektroden die Form von wesentlich rundförmigen, rotierbaren Metallscheiben (7) haben, wobei die Bodenoberfläche Weideland ist und die nachgewiesenen und behandelten Exkremente Rinderurin sind und wobei die zumindest eine Substanz einen Nitrifikationsinhibitor und/oder einen Ureaseinhibitor umfasst.

2.  Eine Vorrichtung entsprechend Anspruch 1, wobei die Bodenoberfläche Weideland ist und das Erkennungsmittel adaptiert ist, das Vorhandensein von Urin auf der Bodenoberfläche (im Untergrund) durch Messen der lokalisierten Leitfähigkeit oder Resistivität der Erde der Bodenoberfläche (oder des Untergrunds) und/oder Veränderungen in der Leitfähigkeit oder Resistivität der Erde nachzuweisen.

3.  Eine Vorrichtung entsprechend Anspruch 2, wobei das Erkennungsmittel Folgendes umfasst:

    a) zwei äußere Elektroden in Kontakt mit der Bodenoberfläche
    b) zumindest zwei innere Elektroden in Kontakt mit der Bodenoberfläche, wobei die genannten zumindest zwei inneren Elektroden zwischen den beiden äußeren Elektroden positioniert sind und mit den beiden äußeren Elektroden elektrisch verbunden sind,
    c) eine Stromquelle zur Bereitstellung eines elektrischen Stroms, der zwischen den beiden äußeren Elektroden (und somit durch die zumindest zwei inneren Elektroden) fließt,
    d) Mittel zum Messen der elektrischen Spannung zwischen den zumindest zwei inneren Elektroden,
    e) Kontrollmittel zur Analyse der elektrischen Spannung zwischen den zumindest zwei inneren Elektroden, wobei die lokalisierte Leitfähigkeit und/oder Resistivität der Bodenoberfläche (oder des Untergrunds) bestimmt werden kann und demzufolge Bereiche, auf den Urin hinterlassen wurde, identifiziert und/oder lokalisiert werden können,
    wobei die Anordnung und Konstruktion derart sind, dass das Kontrollmittel danach das Behandlungsmittel anweist, Bereiche, auf denen hinterlassener Urin festgestellt wurde, automatisch mit der/den Substanz/en zu behandeln.

4.  Eine Vorrichtung entsprechend Anspruch 1, wobei zu den rotierbaren Metallscheiben Zacken um den Rand gehören, um in die Bodenoberfläche einzugreifen und/oder in diese einzudringen.

5.  Eine Vorrichtung entsprechend einem der Ansprüche 1 bis 5, wobei die Vorrichtung ein Fahrzeug mit Rädern ist oder adaptiert wurde, um hinter einem Fahrzeug mit Rädern gezogen zu werden.

6.  Eine Vorrichtung entsprechend Anspruch 6, wobei das Fahrzeug durch Fernbedienung betrieben wird.

7.  Eine Vorrichtung entsprechend Anspruch 6, wobei das Fahrzeug automatisiert und selbstfahrend ist.

8.  Eine Vorrichtung entsprechend einem der Ansprüche 6 bis 8, wobei zum Fahrzeug Nähesensoren gehören.

9.  Eine Vorrichtung entsprechend einem der Ansprüche 1 bis 8, wobei zur Vorrichtung ein Reservoir jeder der zumindest einen Substanz gehört, wobei jedes des/der genannte/n Reservoir/e mit einem Verteilungsmittel ausgestattet ist, damit die zumindest eine Substanz auf jeden Bereich der Bodenoberfläche aufgetragen wird, auf dem durch das Erkennungsmittel hinterlassene Exkremente nachgewiesen wurden.

10. Eine Vorrichtung entsprechend Anspruch 9, wobei zum Verteilungsmittel eine oder mehrere Sprühdüsen gehören, die adaptiert wurden, um die zumindest eine Substanz auf jeden Bereich der Bodenoberfläche zu sprühen, wo durch das Erkennungsmittel hinterlassene Exkremente nachgewiesen wurden.

11. Ein Verfahren zum Erkennen eines Bereichs einer Bodenoberfläche (oder eines Untergrunds), auf dem Exkremente hinterlassen wurden, und nachfolgender Behandlung des Bereichs mit einer Substanz/mit Substanzen, **dadurch gekennzeichnet, dass** zum genannten Verfahren der Schritt der Nutzung der Vorrichtung entsprechend einem der Ansprüche 1 bis 10 gehört.

12. Ein V erfahren entsprechend Anspruch 11 zum Erkennen und Behandeln von kürzliche hinterlassenem Rinderurin, d.h. innerhalb von 72 Stunden nach dem Hinterlassen.

# EP 3 066 466 B1

**Revendications**

1. Appareil pouvant se déplacer sur une surface au sol, ledit appareil comprenant :

   a) un dispositif de détection (3) pour la détection d'une zone de la surface au sol (ou d'une surface sous le sol) sur laquelle one été déposées des excréta,
   b) un dispositif de traitement (6, 9) pour le traitement de la zone avec au moins une substance,
   ledit dispositif de détection étant adapté pour détecter la présence d'excréta sur la surface au sol (ou la surface sous le sol) en mesurant un flux de courant électrique sur une pluralité d'électrodes au contact de la surface du sol,
   **caractérisé en ce que**
   lesdites électrodes se présentent sous forme de disques métalliques substantiellement circulaires et rotatifs (7), la surface au sol étant un pâturage, les excréta détectés et traités étant de l'urine de bétail, et l'au moins une substance comprenant un inhibiteur de nitrate et un inhibiteur d'uréase.

2. Appareil selon la revendication 1, la surface au sol étant un pâturage et le dispositif de détection étant adapté pour détecter la présence d'urine sur la surface du sol (ou la surface sous le sol) en mesurant la conductivité ou la résistivité localisées du sol de la surface du sol (ou de la surface sous le sol) et/ou des variations de la conductivité ou de la résistivité du sol.

3. Appareil selon la revendication 2, le dispositif de détection comprenant :

   a) deux électrodes extérieures au contact de la surface du sol,
   b) au moins deux électrodes intérieures au contact de la surface du sol, lesdites au moins deux électrodes intérieures étant positionnées entre les deux électrodes extérieures, et étant connectées électriquement aux deux électrodes intérieures,
   c) une source d'alimentation assurant la fourniture d'un courant électrique s'écoulant entre les deux électrodes extérieures (et, par conséquent, à travers les au moins deux électrodes intérieures),
   d) un dispositif de mesure du potentiel électrique entre les au moins deux électrodes intérieures,
   e) un dispositif de contrôle pour analyser le potentiel électrique entre les au moins deux électrodes intérieures, la conductivité ou la résistivité localisées de la surface du sol (ou de la surface sous le sol) pouvant être déterminée, et, en conséquence, toutes les zones sur lesquelles un dépôt d'urine a eu lieu pouvant être identifiées et/ou localisées,
   l'agencement et la structure étant tels que le dispositif de contrôle commande au dispositif de traitement de procéder au traitement automatique des zones possédant les substances sur lesquelles a été déterminée la présence d'un dépôt d'urine.

4. Appareil selon la revendication 1, les disques métalliques rotatifs étant dotés de pointes sur leur pourtour pour engager la surface du sol et/ou pénétrer dans celle-ci.

5. Appareil selon une quelconque des revendications 1 à 5, l'appareil étant un véhicule à roues, ou étant adapté pour être remorqué par un véhicule à roues.

6. Appareil selon la revendication 6, le véhicule étant actionné par un dispositif de télécommande.

7. Appareil selon la revendication 6, le véhicule étant automatisé et automoteur.

8. Appareil selon une quelconque des revendications 6 à 8, le véhicule comprenant des capteurs de proximité.

9. Appareil selon une quelconque des revendications 1 à 8, l'appareil comprenant un stock de chacune de l'au moins une substance, chacun desdits stocks étant muni d'un dispositif de distribution pour permettre l'application d'au moins une substance dans chaque zone de la surface au sol où un dépôt d'excréta a été détecté par le dispositif de détection.

10. Appareil selon la revendication 9, le dispositif de distribution comprenant une ou plusieurs buses de pulvérisation adaptées pour pulvériser l'au moins une substance sur chaque zone de la surface au sol où un dépôt d'excréta a été détecté par le dispositif de détection.

11. Méthode de détection d'une zone d'une surface au sol (ou d'une surface sous le sol) sur laquelle ont été déposées des excréta, puis de traitement de la zone avec une (des) substance(s),
**caractérisée par le fait que** ladite méthode comprend l'étape d'utilisation de l'appareil selon une quelconque des revendications 1 à 10.

12. Méthode selon la revendication 11, pour la détection et le traitement d'un dépôt récent d'urine de bétail, à savoir dans les 72 heures suivant le dépôt.

Fig 1

Fig 2

Fig 3

Fig 4

Fig 5a

Fig 5b

10a

+24v
v1
v2
Gnd
v1
v2
+24v
v1
v2
Gnd
v1
v2
+24v
v1
v2
Gnd

7

7

7

10b

Fig 5c

11

Fig 6

Fig 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- NZ 582798
- NZ 254659
- NZ 506883

- WO 0219809 A1
- US 5033397 A
- WO 2006132549 A

**Non-patent literature cited in the description**

- **DI, H.J. ; CAMERON, K. C.** The use of a nitrification inhibitor, dicyandiamide (DCD), to decrease nitrate leaching and nitrous oxide emissions in a simulated grazed and irrigated grassland. *Soil Use and Management,* 2002, vol. 18, 395-403

- **DAWAR, K. ; ZAMAN, M ; ROWARTH, J.S. ; TURNBULL, M.H.** Applying urea with urease inhibitor (N-(n-butyl) thiophosphoric triamide) in fine particle application improves nitrogen uptake in ryegrass (Lolium perenne L.). *Soil Science and Plant Nutrition,* 2012, vol. 58 (3), 309-318
- **L. RODHE ; E. SALOMON ; M. GILBERTSSON.** Detection of urine patches on grassland with an electromagnetic induction - based sensor. *Proceeding at the NJF-Seminar,* 25 January 2011, vol. 438